# EUROPEAN PATENT SPECIFICATION

(11) **EP 1 013 245 B1**
(45) Date of publication and mention of the grant of the patent: **17.08.2005**
(21) Application number: 99310209.4
(22) Date of filing: 16.12.1999
(51) Int. Cl.: A61F 2/36

(54) **Femoral component**
Femurkomponente
Composant fémoral

(30) Priority: 18.12.1998 GB 9828085
(43) Date of publication of application: 28.06.2000
(73) Proprietor: BENOIST GIRARD SAS, 14200 Hérouville-Saint-Clair (FR)
(72) Inventor: Ling, Robin Sydney Mackwood, Prof., Dittisham, Dartmouth, Devon TQ6 0HB (GB); Gie, Graham Allan, Yeoford, Devon EX17 5EZ (GB); Timperley, Andrew John, St. Leonard's, Exeter EX2 4PA (GB); Storer, John Andrew, 14250 Jouaye Mondaye, Bayeux (FR)
(74) Representative: Bridge-Butler, Alan James

(56) References cited:
- EP-A- 0 038 897
- EP-A- 0 436 317
- EP-A- 0 457 222
- FR-A- 2 580 171
- US-A- 5 653 764

## Description

This invention relates to a femoral component of a replacement hip joint of the "Exeter" type which has a collarless stem including a shoulder and for fixing in a medullary canal by cement.

The "Exeter" type femoral component of the kind shown in British Patent No 1 409 054 is well known and comprises a neck which carries a ball head for cooperation with an acetabular socket. The neck is connected to a tapered collarless stem. Thus there is no collar for resting either on the bone or the cement in the area where the stem joins the neck of the implant. This type of stem has evolved so that the stem can be given a polished finish to help it slide down inside the bone cement and the present invention relates to this type of femoral component.

In certain circumstances it can be difficult to locate the femoral component in the medullary socket with the neck and ball at the precise height and version required. This can be caused by the bone which are structurally imperfect or by difficulties in reaming a suitable cavity. It is particularly pronounced when cavities have to be lined with bone fragments prior to cementing or when there are irregularities and the present invention is intended to overcome some of the difficulties experienced.

It is known from EP-A-0 845 251 to provide a femoral component with a separate proximal component but in this Application the angle of the proximal component with regard to the stem about a proximal/distal axis is fixed and there is no provision for adjustment.

US Patent Specification 4 051 559 also shows the use of a separate proximal component and this is provided to allow it to be placed in position on a cylindrical stem which is intended to be screwed directly into the bone. The proximal component is provided with a collar which is intended to rest against the cut and prepared bone and there is no provision for a stem to slide down inside bone cement as is required by the Exeter type component. Moreover, the angular adjustment about a proximal/distal axis is much too coarse to allow final accurate adjustment with an Exeter type component.

US Patent specification 5,653,764 shows a two-piece modular hip prosthesis which has a stem portion and a neck portion which has a modular or fixed head ball and a neck region which tapers to a wider shoulder portion. The neck portion can be connected to the stem by attachment means which allow a range of relatively angular positions. The stem or the neck portion can include male and female elements respectively and means can be provided for fixing the relatively angular positions by pins or splines. In this construction however the relative positions are determined by the splines or pins themselves so that the number and variation of positions is restricted.

EP 0 436 317 shows a prosthetic bearing assembly comprising a fixation element for attachment to the available anatomy and a substantially circular inner bearing component for location therein. Separate location means are provided which act on the fixation element and the bearing component to locate them with a Vernier adjustment in any one of a number of different relatively circumferential positions. This construction is particularly useful for acetabular cups.

According to the present invention a femoral component of a replacement hip joint has a stem for fixing in cement in a medullary cavity and which has a proximal/distal axis A and a separate proximal component which has a neck to receive a ball head, and including attachment means which include a male element on the stem adapted to co-operate with a female structure on the proximal component, which allows the selection of a number of angularly displaced positions about the proximal/distal axis and adapted to allow fixation of the two parts after insertion of the stem into the medullary cavity causing minimal torsion loads on said stem,
characterised in that said stem and said separate proximal component are each provided with a predetermined number of openings arranged at the same radius about the proximal/distal axis, one ring of openings having one more opening than the other, and a locking member and adapted to enter one of each ring of openings to provide a Vernier coupling.

The use of a Vernier coupling enables a very large number of positions to be used and provides a simple and inexpensive method of manufacture.

Thus, the male element may comprise a boss on the proximal part of the stem and said female structure can be provided by a co-operating cavity to receive the boss.

In one preferred embodiment the boss and cavity are tapered and in another they are cylindrical.

The invention can be performed in various ways and two embodiments will now be described by way of example and with reference to the accompanying drawings in which:
Figure 1 is a diagrammatic part cross-sectional side elevation of a femoral component according to the invention;
Figure 2 is a partial plan view of the construction shown in Figure 1;
Figure 3 is a plan view on the line III-III of Figure 1; and,
Figure 4 is a view similar to Figure 1 of an alternative construction; and,
Figure 5 is a view similar to Figure 4 of another alternative construction.

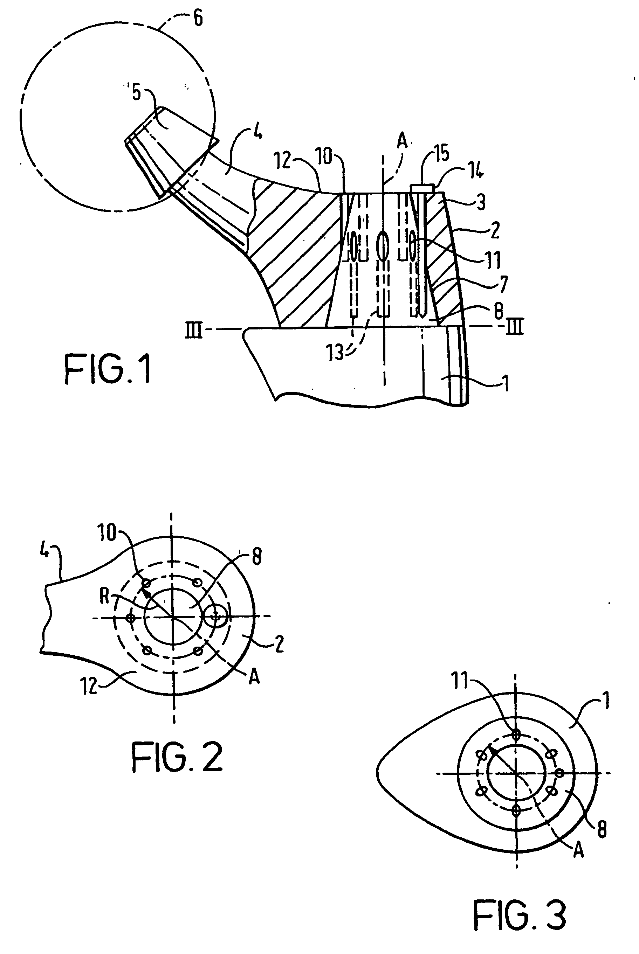

As shown in Figures 1 to 3 the femoral component according to the invention comprises a stem 1 and a proximal component 2 which has a shoulder 3 and a neck 4 with a tapered boss 5 to receive a ball head 6. A Vernier coupling is provided by a ring of openings 10 in the proximal component 2. The openings 10 are arranged on a radius r about a proximal/distal axis A of the stem.

A second ring of openings 11 are provided on the stem 1 and are arranged on the same radius **r** about the same axis A as the openings 10 in the proximal component. These openings 11 extend into the wall of a boss 8 whilst the openings 10 are arranged to open through a proximal surface 12 of the proximal component surrounding the open end of a tapered cavity 7. As will be seen from Figure 1 the distal end of the openings 10 extend into the tapered cavity 7 at a position in line with the proximal ends of the openings 11 in the boss and the distal end 13 of each of the openings 11 is towards the distal end of the boss.

A locating pin 14 having a head 15 is provided which is a push or tight fit in the openings 11. In an alternative embodiment (not shown) the openings 11 are screw threaded and the pin 14 is replaced by a screw which can engage the thread.

The Vernier effect is created in the construction shown in Figure 1 by providing six openings 10 and seven openings 11. This allows small increments of angular adjustment between the proximal component and the stem 1.

If desired more accuracy of adjustment can be arranged by providing more openings 10 and 11 but only six and seven respectively have been shown in the drawings so that they are clear.

If a pin 14 is used then any pressure placed on the femoral component is in a direction of the proximal/distal axis. If a screw is used then any torsional twisting movement of the stem when the proximal component is fitted need only be very light.

In order to fit the femoral component a medullary cavity is prepared in the usual way and the stem 1 is first inserted, being allowed to take up any angular position about a proximal/distal axis as is desired by the surgeon, thus enabling the maximum cement mantle to be achieved. The proximal component is now fitted and the correct angle assessed by the surgeon, the very small increments of angular movement allowing good accuracy. The pin 14 or screw can be lightly inserted and then the femoral component tapped or have pressure applied to ensure that the taper between the boss and the cavity locks. The pin or screw 14 is now driven fully home thus ensuring that the proximal component and the stem are firmly locked together.

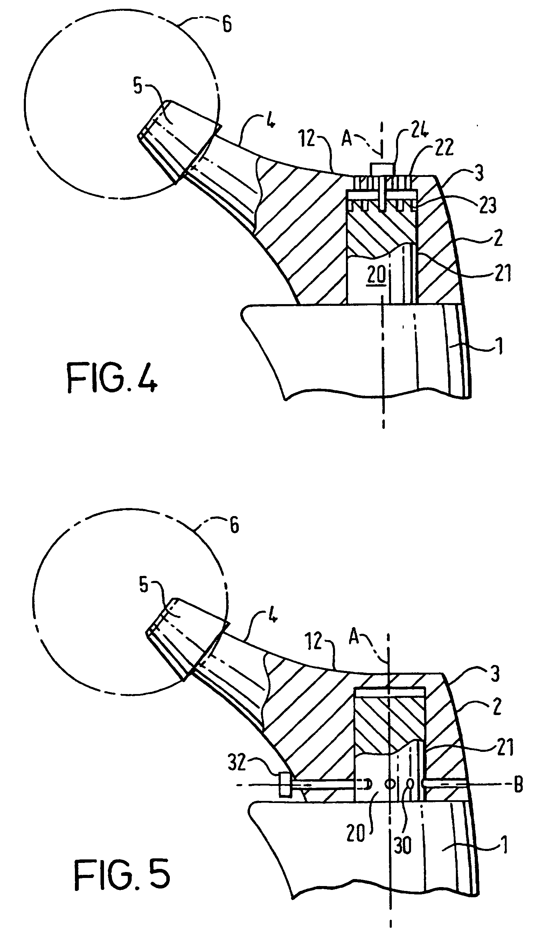

An alternative embodiment is shown in Figure 4 and the same reference numerals are used to indicate similar parts. In this arrangement however a cylindrical boss 20 is employed on the stem 1 and is a close sliding fit in a cylindrical cavity 21 in the proximal component 2. The end of the cavity 21 is closed and the end wall is provided with a ring of openings 22 which are again around a fixed radius based on a proximal/distal axis A. A further ring of openings 23 are provided in the proximal end of the boss 20 which are again on the same radius and about the same axis A. Once again there is one more hole 23 than holes 22 and a Vernier coupling is provided by the insertion of a pin or screw 24 into an appropriate pair of mating holes at the desired angular position.

Figure 5 shows another alternative construction again employing the same reference numerals to indicate similar parts. In this construction a cylindrical boss 20 is again shown but this carries a ring of blind openings 30 which are aligned in a plane B with a second ring of openings 31 provided in the proximal component 2. There are six openings 30 and seven openings 31 and a pin or screw 32 is provided to achieve the Vernier coupling.

This arrangement could also be employed with a tapered boss and cavity of the kind shown in Figure 1.

## Claims

1. A femoral component of a replacement hip joint having a stem (1) for fixing in cement in a medullary cavity and which has a proximal/distal axis A and a separate proximal component (2) which has a neck (4) to receive a ball head (6), and including attachment means which include a male element (8) on the stem (2) adapted to co-operate with a female structure (7) on the proximal component, which allows the selection of a number of angularly displaced positions about the proximal/distal axis (4) and adapted to allow fixation of the two parts after insertion of the stem (1) into the medullary cavity causing minimal torsion loads on said stem (1), **characterised in that** said stem (1) and said separate proximal component (2) are each provided with a predetermined number of openings (10) (11) arranged at the same radius about the proximal/distal axis (A), one ring of openings (10) (11) having one more opening than the other, and a locking member (15) and adapted to enter one of each ring of openings (10) (11) to provide a Vernier coupling.

2. A femoral component as claimed in claim 1 **characterised in that** said male element comprises a boss (8) on the proximal part of said stem (1) and said female structure is provided by a co-operating cavity (7) to receive the boss (8).

3. A femoral component as claimed in claim 2 **characterised in that** said boss (8) and said cavity (7) are tapered.

4. A femoral component as claimed in claim 2 **characterised in that** said boss (8) and said cavity (7) are cylindrical.

5. A femoral component as claimed in any one of the preceding claims **characterised in that** said openings (10) (11) extend in directions parallel to the proximal/distal axis (A).

6. A femoral component as claimed in any one of the preceding claims 1 to 4 **characterised in that** the predetermined openings (10) (11) are aligned in a plane (B) normal to the proximal/distal axis (A).

7. A femoral component as claimed in any one of the preceding claims **characterised in that** the locking member is a pin (15).

## Patentansprüche

1. Femorale Komponente eines Ersatz-Hüftgelenks mit einem Schaft (1) zur Fixierung in Zement in einem medullären Hohlraum und der eine proximal/distale Achse A aufweist, und einer getrennten proximalen Komponente (2), die einen Hals (4) aufweist, um einen Kugelkopf (6) aufzunehmen, und einschließend Befestigungsmittel, die ein Einsteckelement (8) auf dem Schaft (2) einschließen, das angepasst ist, um mit einer Aufnahmestruktur (7) auf der proximalen Komponente zusammenzuwirken, welche die Auswahl einer Anzahl von winkelversetzten Stellungen um die proximal/distale Achse (A) herum zulässt und angepasst ist, um eine Hefestigung der zwei Teile nach einem Einsetzen des Schaftes (1) in den medullären Hohlraum zuzulassen, die auf dem Schaft (1) minimale Torsionsbelastungen verursacht, **dadurch gekennzeichnet, dass** der Schaft (1) und die getrennte proximale Komponente (2) jeweils mit einer vorbestimmten Anzahl von Öffnungen (10) (11) versehen sind, die an demselben Radius um die proximal/distale Achse (A) herum angeordnet sind, wobei ein Ring von Öffnungen (10) (11) eine Öffnung mehr als der andere aufweist, sowie ein Verriegelungselement (15) und angepasst, um in eine von jedem Ring von Öffnungen (10) (11) einzudringen, um eine Vernier-Kupplung bereit zu stellen.

2. Femorale Komponente nach Anspruch 1, **dadurch gekennzeichnet, dass** das Einsteckelement einen vorsprung (8) auf dem proximalen Teil des Schaftes (1) umfasst und die Aufnahmestruktur von einer zusammenwirkenden Ausnehmung (7) zur Aufnahme des Vorsprungs (8) bereit gestellt wird.

3. Femorale Komponente nach Anspruch 2, **dadurch gekennzeichnet, dass** der Vorsprung (8) und die Ausnehmung (7) verjüngt sind.

4. Femorale Komponente nach Anspruch 2, **dadurch gekennzeichnet, dass** der Vorsprung (8) und die Ausnehmung (7) zylindrisch sind.

5. Femorale Komponente nach einem der vorangehenden Ansprüche, **dadurch gekennzeichnet, dass** sich die Öffnungen (10)(11) jeweils in einer Richtung parallel zur proximal/distalen Achse (A) erstrecken,

6. Femorale Komponente nach einem dur vorangehenden Ansprüche 1 bis 4, **dadurch gekennzeichnet, dass** die vorbestimmten öffnungen (10) (11) in einer zur proximal/distalen Achse (A) senkrechten Ebene (8) miteinander fluchten.

7. Femorale Komponente nach einem der vorangehenden Ansprüche, **dadurch gekennzeichnet, dass** das Verriegelungselement ein Bolzen (15) ist.

## Revendications

1. Composant fémoral pour une articulation de remplacement de la hanche, comportant une tige (1) pour la fixation dans du ciment dans une cavité médullaire, et qui comporte un axe proximal/distal A, et un composant proximal séparé (2) qui comporte un col (4) pour recevoir une tête à rotule (6), et comprenant des moyens de fixation qui comprennent un élément mâle (8) sur la tige (2), adapté pour coopérer avec une structure femelle (7) sur le composant proximal, permettant la sélection d'un certain nombre de positions décalées de façon angulaire autour de l'axe proximal/distal (4), et adaptés pour permettre la fixation des deux parties après l'insertion de la tige (1) dans la cavité médullaire, de façon à provoquer des charges de torsion minimales sur ladite tige (1), **caractérisé en ce que** ladite tige (1) et ledit composant proximal séparé (2) sont chacun munis d'un nombre prédéterminé d'ouvertures (10) (11) configurées avec le même rayon autour de l'axe proximal/distal (A), un anneau d'ouvertures (10) (11) comportant une ouverture de plus que l'autre, et un élément de verrouillage (15), et adapté pour entrer dans chacun des anneaux d'ouvertures (10) (11) de façon à produire un accouplement à Vernier.

2. Composant fémoral selon la revendication 1, **caractérisé en ce que** ledit élément mâle comprend une protubérance (8) sur la partie proximale de ladite tige (1), et **en ce que** ladite structure femelle est constituée par une cavité coopérante (7) pour recevoir la protubérance (8).

3. Composant fémoral selon la revendication 2, **caractérisé en ce que** ladite protubérance (8) et ladite cavité (7) sont coniques.

4. Composant fémoral selon la revendication 2, **caractérisé en ce que** ladite protubérance (8) et ladite cavité (7) sont cylindriques.

5. Composant fémoral selon l'une quelconque des revendications précédentes, **caractérisé en ce que** lesdites ouvertures (10) (11) s'étendent dans des directions parallèles à l'axe proximal/distal (A).

6. Composant fémoral selon l'une quelconque des revendications 1 à 4 qui précèdent, **caractérisé en ce que** les ouvertures prédéterminées (10) (11) sont alignées dans un plan (B) normal à l'axe proximal/distal (A).

7. Composant fémoral selon l'une quelconque des revendications précédentes, **caractérisé en ce que** l'élément de verrouillage est une broche (15).
